# EUROPEAN PATENT SPECIFICATION

(11) **EP 3 058 917 B1**
(45) Date of publication and mention of the grant of the patent: **23.05.2018**
(21) Application number: 16164980.1
(22) Date of filing: 12.04.2016
(51) Int. Cl.: A61F 13/49, A61F 13/15

(54) **METHOD OF MANUFACTURING ABSORBENT ARTICLE**
VERFAHREN ZUR HERSTELLUNG EINES SAUGFÄHIGEN ARTIKELS
PROCÉDÉ DE FABRICATION D'UN ARTICLE ABSORBANT

(30) Priority: 08.04.2016 JP 2016078477
(43) Date of publication of application: 24.08.2016
(73) Proprietor: Unicharm Corporation, Ehime 799-0111 (JP)
(72) Inventor: Morizane, Naoto, Kanonji-shi, Kagawa 769-1602 (JP); Seto, Hidenao, Kanonji-shi, Kagawa 769-1602 (JP)
(74) Representative: Dolleymores

(56) References cited:
- EP-A1- 2 260 813
- WO-A1-2009/146307
- JP-A- 2001 212 174
- JP-A- 2013 013 466
- US-A- 5 308 345

## Description

### Technical Field

The present invention relates to a method for producing an absorbent article.

### Background Art

Absorbent articles such as disposable diapers are known. In order to increase fitting onto the human body when worn, such absorbent articles are provided with expandable-and-shrinkable members that are expandable and shrinkable in an expansion-and-shrink direction. Such expandable-and-shrinkable members includes waist gather sections provided surrounding essentially the entire abdominal side and back side, or tape-like waist gather sections provided on the back side.

Apparatuses for forming tape-like waist gather sections on absorbent articles include the production apparatus of Patent Literature 1 and the widening mechanism of Patent Literature 2, for example. In the production apparatus of Patent Literature 1, an expandable-and-shrinkable member (elastic member) is held piercing the same with pin members while suctioning both ends in the expansion-and-shrink direction. Next, the held expandable-and-shrinkable member is moved toward the web surface while expanding in the expansion-and-shrink direction. The expanded expandable-and-shrinkable member is pressed against the attachment position of the web surface, and suction is terminated and the pin members are removed to attach the expandable-and-shrinkable member onto the web surface. The widening mechanism of Patent Literature 2 also has essentially the same construction.

### Citation List

### Patent Literature

[Patent Literature 1] Japanese Laid-Open Patent Publication No. 2013-13466
[Patent Literature 2] Japanese Laid-Open Patent Publication No. 2001-212174

Further prior art arrangements are known from WO 2009/146307, US 5308345 and JP 2001212174.

### SUMMARY OF INVENTION

### Technical Problem

Incidentally, in the technology of Patent Literatures 1 and 2, the entire expandable-and-shrinkable member is expanded essentially uniformly and the expandable-and-shrinkable member is attached onto the web surface. The attached expandable-and-shrinkable member then shrinks and functions as a gather section. When the expandable-and-shrinkable member shrinks, the section (surface) on the side not in contact with the web can shrink directly, but the section (surface) on the side in contact with the web must shrink while deforming the web. Thus, the section (surface) on the side in contact with the web is limited in its shrink compared to the section (surface) on the side not in contact with the web. As a result, the laminate of the web and the expandable-and-shrinkable member has internal stress, resulting in concavity on the expandable-and-shrinkable member side, or in other words, it tends to warp around to the expandable-and-shrinkable member side.

Moreover, with the technologies in Patent Literatures 1 and 2, an adhesive is coated onto the expandable-and-shrinkable member and/or web, and the expandable-and-shrinkable member is pressed against the web to attach the elastic member to the web through the adhesive. During this time, since both end sections of the expandable-and-shrinkable member are not always sufficiently pressed against the web, it is sometimes impossible to adequately achieve bonding between the both end sections of the expandable-and-shrinkable member and the web with the adhesive.

When the expandable-and-shrinkable member as a whole is expanded essentially evenly and bonded to the web, and the expandable-and-shrinkable member is shrunk, the internal stress that tends to cause the expandable-and-shrinkable member to warp around, and the lack of sufficient bonding of the adhesive at both ends of the expandable-and-shrinkable member, can potentially result in detachment of the bonding at the both ends of the expandable-and-shrinkable member and the web in the subsequent process.

Furthermore, even if bonding is maintained at the both ends where detachment can potentially occur, the weakened bonding may result in deformation to form an unnatural irregular shape that differs from a largely regular wrinkled form at the center section of the expandable-and-shrinkable member. When such an irregular shape is formed, the relative rigidity increases at those sections, potentially lowering the wearing comfort. Moreover, such an irregular shape has a poor visual appearance from the skin side, for example, and can potentially impair the appearance of the product.

It is an object of the invention to provide a method for producing an absorbent article that can improve the bonding performance between the expandable-and-shrinkable member and web at both ends of the expandable-and-shrinkable member, while also improving the product appearance.

### Solution to Problem

A method for producing an absorbent article according to the present invention is as follows.
(1) A method for producing an absorbent article as recited by Claim 1.

In the method for producing an absorbent article, when the expandable-and-shrinkable member is attached to the first continuous sheet (web), the degree of expanding increases at the center section of the expandable-and-shrinkable member and the degree of expanding decreases at the both end sections of the expandable-and-shrinkable member. As a result, internal stress by which the surface on the expandable-and-shrinkable member side of the laminate of the expandable-and-shrinkable member and the first continuous sheet tends toward concavity, i.e. internal stress by which it tends to warp around to the expandable-and-shrinkable member side, can be reduced at the both end sections of the expandable-and-shrinkable member. In addition, when the first continuous sheet and the second continuous sheet are bonded, while the both end sections of the expandable-and-shrinkable member are covered by the cover sheet. In this case, the end sections of the expandable-and-shrinkable member covered by the cover sheet have increased thickness in the thickness direction compared to when they are not covered by the cover sheet, and they are therefore pressed more forcefully when they are bonded. In other words, the bonded sections between the both end sections of the expandable-and-shrinkable member and the first sheet are compressed more forcefully. Consequently, bonding between the both end sections of the expandable-and-shrinkable member and the first continuous sheet, which can potentially become weakened, can be reinforced by the compression. By these effects, it is possible to prevent detachment of bonding between the both end sections of the expandable-and-shrinkable member and the first continuous sheet. In addition, even if the aforementioned unnatural irregular shape or the like is formed, it is possible to minimize the degree of irregularities by the compression. Moreover, the cover sheet makes it difficult for the irregular shape to be visible from the exterior. According to this producing method, therefore, the bonding performance between the expandable-and-shrinkable member and the first continuous sheet at the both end sections of the expandable-and-shrinkable member is improved, and generation of irregular shapes and the like is minimized, while even if irregular shapes and the like are generated, the appearance of the product can be improved. Of the first sheet and second sheet, one is the front sheet and the other is the back sheet. The order of carrying out the cover sheet bonding step, expandable-and-shrinkable member bonding step and integral bonding step may be as desired.

The method for producing an absorbent article according to the present invention may be (2) the method for producing an absorbent article according to the above (1), wherein the expandable-and-shrinkable member bonding step includes a step of coating a hot-melt adhesive onto at least one of the first sheet and the expandable-and-shrinkable member.

In the method for producing an absorbent article, the hot-melt adhesive is present between the irregular shape or the like and the first sheet, so that the irregular shape can be rendered poorly visible. This allows the appearance of the product to be improved.

The method for producing an absorbent article according to the present invention may be (3) the method for producing an absorbent article according to the above (1) or (2), wherein the cover sheet bonding step includes a step of bonding the cover sheet to the second continuous sheet or the first continuous sheet by a heat sealing method.

In this producing method, the cover sheet bonded by a heat sealing method and the irregular shape or the like overlap, making it possible to render the irregular shape or the like poorly visible.

The method for producing an absorbent article according to the present invention may be (4) the method for producing an absorbent article according to the above (1) or (2), wherein the cover sheet bonding step includes a step of bonding the cover sheet to the second continuous sheet or the first continuous sheet by an ultrasonic method.

In this producing method, the cover sheet bonded by an ultrasonic method and the irregular shape or the like overlap, making it possible to render the irregular shape or the like poorly visible.

The method for producing an absorbent article according to the present invention may also be (5) the method for producing an absorbent article according to any one of the above (1) to (4), further comprising a step of forming embossed sections along a lengthwise direction extending through both end sections of the expandable-and-shrinkable member.

In this producing method, embossed sections are formed along the lengthwise direction extending through the both end sections of the expandable-and-shrinkable member, and overlap the irregular shape or the like, making it possible to render the irregular shape or the like poorly visible.

The method for producing an absorbent article according to the present invention may also be (6) the method for producing an absorbent article according to any one of the above (1) to (5), wherein the cover sheet bonding step includes a step of bonding the cover sheet formed of a colored or opaque material to the second continuous sheet or the first continuous sheet.

In this producing method, the cover sheet is colored or opaque, making it difficult for the irregular shape or the like to be seen. Here, opaque means a transmittance of no greater than 90% for visible light rays (for example, 550 nm wavelength).

The method for producing an absorbent article according to the present invention may also be (7) the method for producing an absorbent article according to the above (6), wherein the cover sheet bonding step includes a step of bonding the cover sheet formed of a material of the same color as the expandable-and-shrinkable member to the second continuous sheet or the first continuous sheet.

In this producing method, the cover sheet has the same color as the expandable-and-shrinkable member, making it possible to render the irregular shape or the like poorly visible.

Since the cover sheet bonding step includes a step of situating a pair of leak resistant walls extending along the machine direction and separated in the widthwise direction of the first sheet or the second sheet on the first sheet or the second sheet in such a manner that edges of the leak resistant walls in the machine direction cover the both end sections as the cover sheet, the edges of the leak resistant walls in the machine direction are formed in such a manner that they cover the both end sections of the expandable-and-shrinkable member, and therefore the edges of the leak resistant walls can function as the cover sheet. In other words, the leak resistant walls make it possible to easily render the irregular shape or the like poorly visible.

### Advantageous Effects of Invention

The present invention can provide a method for producing for an absorbent article that can improve the bonding performance between the expandable-and-shrinkable member and web at both end sections of the expandable-and-shrinkable member, while also improving the product appearance.

### Brief Description of Drawings

Fig. 1 is a diagram showing an absorbent article according to an embodiment.
Fig. 2 is a diagram showing an absorbent article according to the embodiment.
Fig. 3 is a schematic overview of a production apparatus for an absorbent article according to the embodiment.
Fig. 4 is a schematic overview of a widening apparatus for an absorbent article according to the embodiment.
Fig. 5 is a schematic overview of a folding apparatus for an absorbent article according to the embodiment.
Fig. 6 is a diagram illustrating a method for producing an absorbent article according to the embodiment.
Fig. 7 is a diagram illustrating a method for producing an absorbent article according to the embodiment.
Fig. 8 is a diagram illustrating a method for producing an absorbent article according to the embodiment.
Fig. 9 is a diagram illustrating a method for producing an absorbent article according to the embodiment.
Fig. 10 is a diagram illustrating a method for producing an absorbent article according to the embodiment.
Fig. 11 is a diagram illustrating a method for producing an absorbent article according to the embodiment.
Fig. 12 is a diagram showing an absorbent article according to another embodiment.
Fig. 13 is a diagram showing an absorbent article according to yet another embodiment.

### Description of Embodiments

An absorbent article according to the embodiment will now be explained with reference to the attached drawings, using a tape-type (open-type) disposable diaper as an example. However, the types and uses of the absorbent article of the present invention are not limited to this example, and the present invention may be applied to other absorbent articles such as panty-type disposable diapers, for example, without departing from the scope of the subject matter of the present invention.

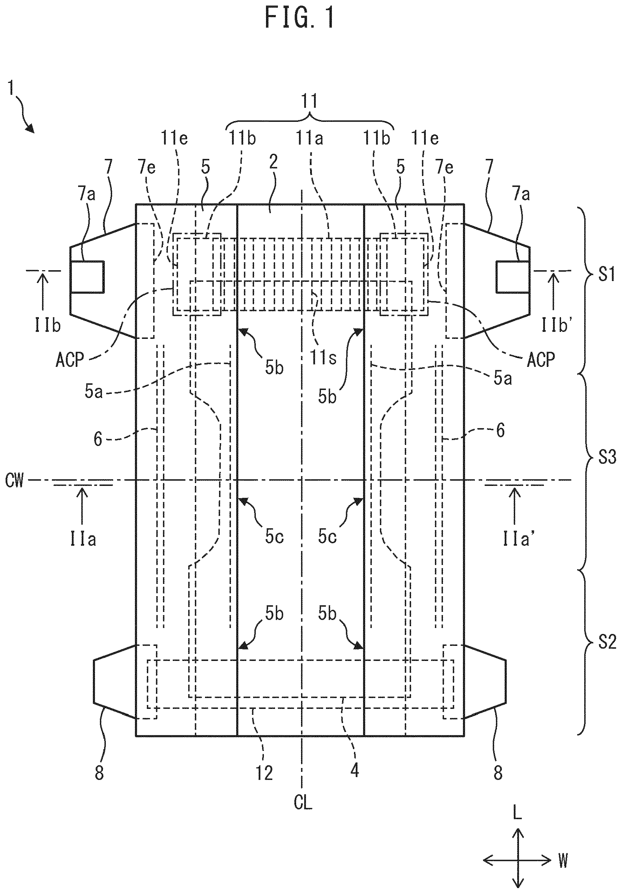

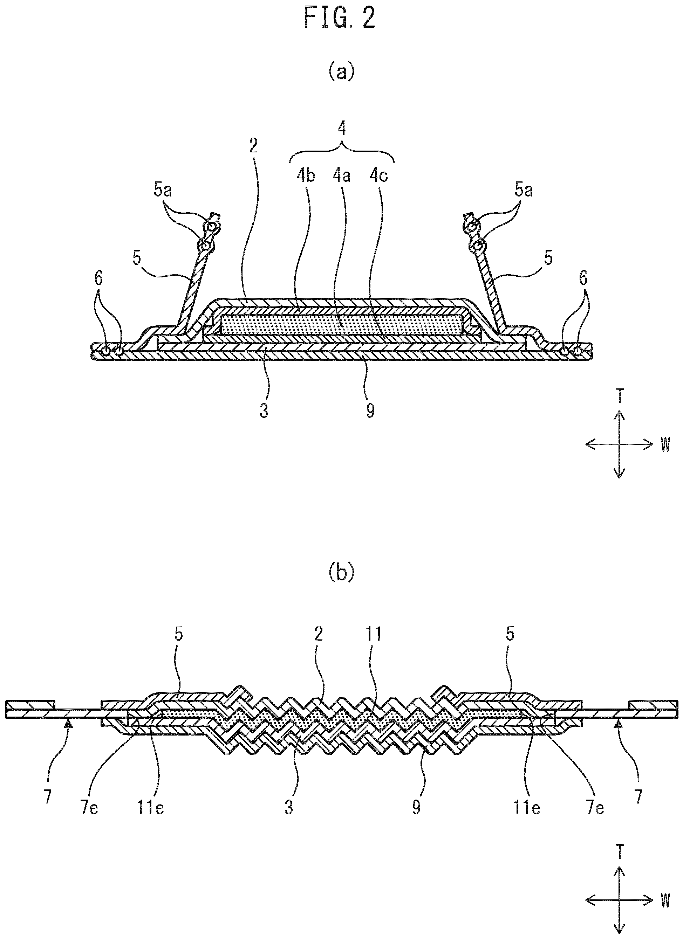

Fig. 1 and Fig. 2 are diagrams showing an absorbent article 1 (disposable diaper), and specifically, Fig. 1 shows a plan view of the absorbent article 1 in the deployed, spread-out state, Fig. 2(a) is a cross-sectional view along line IIa-IIa' of Fig. 1, and Fig. 2(b) is a cross-sectional view along line IIb-IIb' of Fig. 1. However, Fig. 2(b) shows the absorbent article in the natural state, i.e. a state in which the expandable-and-shrinkable member 11 is shrunk, as described later. The absorbent article 1 has a lengthwise direction L, a widthwise direction W perpendicular to the lengthwise direction L, and a thickness direction T perpendicular to the lengthwise direction L and the widthwise direction W, and also has a center axis line CL extending through the center of the widthwise direction W and extending in the lengthwise direction L, and a center axis line CW extending through the center of the lengthwise direction L and extending in the widthwise direction W.

The absorbent article 1 also has, in the lengthwise direction L, a back girth region S1 corresponding to the girth around the back side of the wearer, an abdominal girth region S2 corresponding to the girth around the abdominal side of the wearer, and a groin region S3 located between the back girth region S1 and the abdominal girth region S2, corresponding to the groin of the wearer. It is worn as a diaper by connecting both ends in the widthwise direction W of the back girth region S1 and both ends in the widthwise direction W of the abdominal girth region S2. The groin region S3 may be narrowed in the widthwise direction W.

Throughout the present description, unless otherwise specified, the simple phrase "as plane view" will be used to mean that the absorbent article 1 in the deployed spread-out flat state is viewed from the upper side in the thickness direction. The terms "skin side" and "non-skin side" refer, respectively, to the side relatively near the skin side and the side relatively away from the skin side of the wearer, in the thickness direction of the absorbent article, when the wearer of the absorbent article has worn the absorbent article. Also, the direction toward the center axis line CL is the direction on the inner side in the widthwise direction W, and the direction receding from the center axis line CL is the direction on the outer side in the widthwise direction W. The direction toward the center axis line CW is the direction on the inner side in the lengthwise direction L, and the direction receding from the center axis line CW is the direction on the outer side in the lengthwise direction L.

The absorbent article 1 comprises a front sheet 2, a back sheet 3 and an absorbent body 4. However, the lengthwise direction, widthwise direction and thickness direction of the front sheet 2, back sheet 3 and absorbent body 4 all match the lengthwise direction L, widthwise direction W and thickness direction T of the absorbent article 1. Thus, even for the front sheet 2, back sheet 3 and absorbent body 4, the lengthwise direction L, widthwise direction W and thickness direction T are each used for the lengthwise direction, widthwise direction and thickness direction, and the skin side and non-skin side are each used for the side relatively near and the side relatively away from the skin side of the wearer, in the thickness direction.

The front sheet 2 is a liquid-permeable sheet situated on the skin side of the wearer. The front sheet 2 may be any desired liquid-permeable sheet, such as, for example, a liquid-permeable nonwoven fabric or woven fabric, a liquid-permeable hole-formed synthetic resin film, or a composite sheet thereof. The back sheet 3 is a liquid-impermeable sheet situated on the non-skin side of the wearer. The back sheet 3 may be any desired liquid-impermeable sheet, such as, for example, a liquid-impermeable nonwoven fabric or synthetic resin film, a composite sheet thereof, an SMS nonwoven fabric, or the like. The absorbent body 4 is a fluid-absorbing and liquid-retaining material situated between the front sheet 2 and the back sheet 3, and for this embodiment, it includes an absorbent body core 4a and core wraps 4b, 4c that subsume the absorbent body core 4a. The absorbent body 4 may be pulp fiber, synthetic fiber, absorbing polymer or the like. The absorbent body 4 are bonded to each of the front sheet 2 and back sheet 3 with an adhesive, the front sheet 2 and back sheet 3 being bonded by an adhesive at their peripheries. The adhesive used for bonding between the front sheet 2, absorbent body 4 and back sheet 3 may be a known material that is commonly used in absorbent articles 1, such as a thermoplastic adhesive.

The absorbent article 1 further comprises a pair of leak resistant walls 5, 5, leg expandable-and-shrinkable members 6, 6 and an outer sheet 9. The pair of leak resistant walls 5, 5 are a pair of side section sheets that extend along the lengthwise direction L so as to cover the surfaces of both sides in the widthwise direction W of the front sheet 2, and they are disposed across a distance in the widthwise direction W. The pair of leak resistant walls 5, 5 have their fixed ends at the edges on the outer sides in the widthwise direction W fixed on the surface of both sides in the widthwise direction W of the front sheet 2, and have their free ends forming gather sections that are expandable at the edges on the inner side in the widthwise direction W, also including folded rising sections 5c that are able to rise up in the thickness direction T. Near each of the free ends of the rising sections 5c, 5c of the pair of leak resistant walls 5, 5, there are situated two linear elastic solids 5a such as rubber, for example, extending along the lengthwise direction L. Each elastic solid 5a shrinks the free end of the leak resistant wall 5, thereby causing the pair of leak resistant walls 5, 5 to expand and rise. The pair of leak resistant walls 5, 5 each further include, at both ends in the lengthwise direction L of the rising sections 5c, a joint section 5b extending in the lengthwise direction L, where the rising section 5c is connected to the front sheet 2. The joint sections 5b cover both end sections ACP, ACP of an expandable-and-shrinkable member 11, described below. The leg expandable-and-shrinkable member 6 is an elastic material such as rubber that expands in the lengthwise direction L of each of both sides in the widthwise direction W of the groin region S3 that contacts with the femoral region of the wearer. The outer sheet 9 reinforces the back sheet 3 on the non-skin side of the back sheet 3, improving the hand feel, and being mutually connected to the perimeter sections of the pair of leak resistant walls 5, 5 while bonded to the non-skin side of the back sheet 3. There are no particular restrictions on the materials for the leak resistant walls 5 and the outer sheet 9, and examples include the material for the front sheet 2, for the leak resistant walls 5, and the material for the back sheet 3, for the outer sheet 9.

The absorbent article 1 further comprises an expandable-and-shrinkable member 11 and a pair of protrusion sections 7 in the back girth region S1. The expandable-and-shrinkable member 11 is a sheet that is expandable and shrinkable in an expansion-and-shrink direction, and is disposed between the pair of protrusion sections 7, 7 so as to be along the expansion-and-shrink direction in the widthwise direction W, i.e. expandable and shrinkable in the widthwise direction W, functioning as a waist gather, for example. The expandable-and-shrinkable member 11 is attached using an adhesive to any location of the surface on the skin side of the front sheet 2, the surface on the non-skin side of the back sheet 3, and the region between the front sheet 2 and the back sheet 3, in the back girth region S1. The expandable-and-shrinkable member 11 is also disposed either partially overlapping or without any overlapping with the absorbent body 4, as plane view. For this embodiment, the expandable-and-shrinkable member 11 is disposed between the front sheet 2 and the back sheet 3 in such a manner that the portion on the inner side in the lengthwise direction L of the expandable-and-shrinkable member 11 overlaps with the absorbent body 4 as plane view.

The expandable-and-shrinkable member 11 also includes a high-expanded region 11a located at the center in the widthwise direction W and low-expanded regions 11b located at both sides in the widthwise direction W of the high-expanded region 11a. The high-expanded region 11a is attached to the front sheet 2 and/or back sheet 3 with the expandable-and-shrinkable member 11 in a widely expanded state, and it undergoes large shrink together with the front sheet 2 and/or back sheet 3 by returning to the natural state after having been expanded, whereby a plurality of wrinkles 11S are formed. The wrinkles 11S extend generally in the lengthwise direction L and are arranged generally in the widthwise direction W. The low-expanded regions 11b are attached to the front sheet 2 and/or back sheet 3 with the expandable-and-shrinkable member 11 in a modestly expanded state, and they shrink moderately together with the front sheet 2 and/or back sheet 3 by returning to the natural state after having been expanded, such that few wrinkles are formed. Thus, the high-expanded region 11a is a region capable of high expansion and shrink, and the low-expanded regions 11b are regions capable of low expansion and shrink. However, on the sides of the low-expanded regions 11b near the border between the high-expanded region 11a and the low-expanded regions 11b, i.e. at the both end sections ACP, ACP of the expandable-and-shrinkable member 11, an irregular concavo-convex pattern is sometimes formed by the effect of the difference in expanding. Such an irregular concavo-convex pattern can potentially lower the appearance of the product. For this embodiment, therefore, even if an irregular concavo-convex pattern is produced, it is hidden because the both end sections ACP, ACP are covered from the skin side by the joint sections 5b of the pair of leak resistant walls 5, 5. Consequently, the pair of leak resistant walls 5, 5 (their joint sections 5b) may be considered to be as a cover sheet covering the both end sections ACP, ACP of the expandable-and-shrinkable member 11. The material of the expandable-and-shrinkable member 11 is not particularly restricted so long as it is expandable and shrinkable, and it may be expandable and shrinkable as a material, or expandable and shrinkable in shape, or expandable and shrinkable by combination with an elastic member.

The pair of protrusion sections 7, 7 are sheets for connection of the back girth region S1 to the abdominal girth region S2 during wear, and they function as side flaps. The pair of protrusion sections 7, 7 are disposed so as to protrude toward both outer sides in the widthwise direction W of the back girth region S1, and are attached with an adhesive at any location of the surface of the skin side of the front sheet 2 (or leak resistant wall 5) on the surface of the non-skin side of the back sheet 3 (or outer sheet 9), and the region between the front sheet 2 (or leak resistant wall 5) and the back sheet 3 (or outer sheet 9), in the back girth region S1. However, the pair of protrusion sections 7, 7 may also be formed at the extended sections of either or both the front sheet 2 (or leak resistant wall 5) and the back sheet 3 (or outer sheet 9). For this embodiment, the protrusion sections 7 are disposed between the leak resistant wall 5 and the outer sheet 9, with separate members from the front sheet 2, leak resistant wall 5, back sheet 3 and outer sheet 9. The protrusion sections 7 comprise connecting tapes 7a for connection with a to-be-connected member 12 (described below) in the abdominal girth region S2 during wear. The connecting tapes 7a may be a hook-and-loop fastener or adhesive tape. The material for the protrusion sections 7 is not particularly restricted, and may be the material of the front sheet 2 or back sheet 3, for example.

The expandable-and-shrinkable member 11 is disposed between the pair of protrusion sections 7, 7. That is, it is disposed in such a manner that the expandable-and-shrinkable member 11 and the pair of protrusion sections 7, 7 overlap in at least sections in the lengthwise direction L. In other words, projection onto the center axis line CL of the edge line 11e along the lengthwise direction L on the outer side in the widthwise direction W of the expandable-and-shrinkable member 11, and projection onto the center axis line CL of the edge line 7e along the lengthwise direction L on the inner side in the widthwise direction W of the protrusion section 7, overlap at least partially. This can improve the fitting property of the absorbent article 1 in the girth region during wear. In the cross-machine direction CD, the expandable-and-shrinkable member 11 and the protrusion sections 7 may be partially overlapping, or they may be non-overlapping.

The absorbent article 1 further comprises a to-be-connected member 12 and a pair of protrusion sections 8 in the abdominal girth region S2. The to-be-connected member 12 is a sheet that is to be connected with the connecting tapes 7a of the pair of protrusion sections 7, 7, and when the connecting tapes 7a are hooks of hook-and-loop fasteners, for example, the to-be-connected member 12 is a loop of a hook-and-loop fastener, and when the connecting tapes 7a are adhesive tapes, the to-be-connected member 12 is a sheet capable of pressure-sensitive adhesion with the adhesive tape. The to-be-connected member 12 is attached with an adhesive at a location on the surface on the non-skin side of the outer sheet 9 in the abdominal girth region S2. The to-be-connected member 12 is also disposed either partially overlapping or without any overlapping with the absorbent body 4, as plane view. For this embodiment, the to-be-connected member 12 is disposed on the surface on the non-skin side of the outer sheet 9, in such a manner that a portion on the inner side in the lengthwise direction L of the to-be-connected member 12 overlaps with the absorbent body 4, as plane view. The pair of protrusion sections 8, 8 are sheets for connection of the back girth region S2 to the abdominal girth region S1 during wear, and they function as side flaps. The pair of protrusion sections 8 are disposed so as to protrude toward both outer sides in the widthwise direction W of the abdominal girth region S2, and are attached with an adhesive at any location of the surface of the skin side of the front sheet 2 (or leak resistant wall 5), the surface of the non-skin side of the back sheet 3 (or outer sheet 9), and the region between the front sheet 2 (or leak resistant wall 5) and the back sheet 3 (or outer sheet 9), in the abdominal girth region S2. However, the pair of protrusion sections 8, 8 may also be formed at the extended sections of either or both the front sheet 2 (or leak resistant wall 5) and the back sheet 3 (or outer sheet 9). For this embodiment, the protrusion sections 8 are disposed between the leak resistant wall 5 and the outer sheet 9, with separate members from the front sheet 2, leak resistant wall 5, back sheet 3 and outer sheet 9. The material for the protrusion sections 8 is not particularly restricted, and may be the material of the front sheet 2 or back sheet 3, for example. The protrusion sections 8 may also be absent.

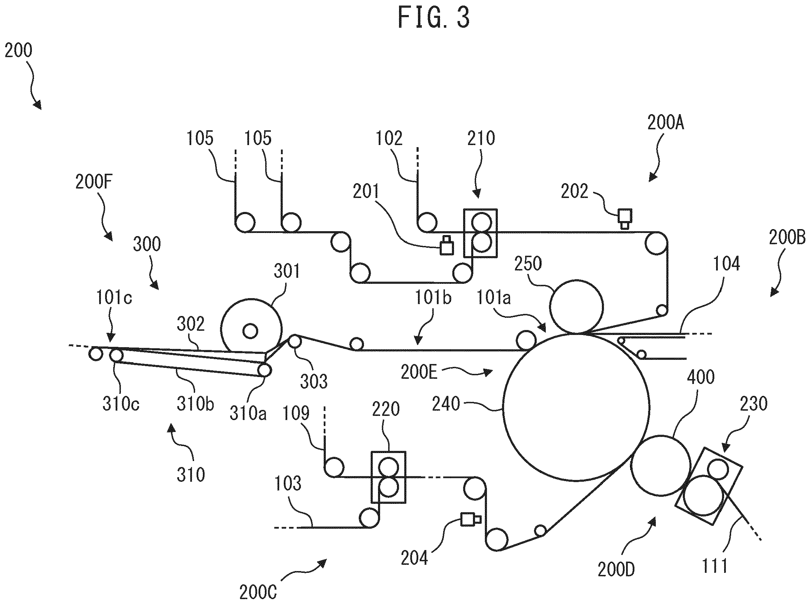

A production apparatus for an absorbent article according to this embodiment will now be described. Fig. 3 shows an example of a configuration of a production apparatus 200 for an absorbent article 1. The production apparatus 200 comprises a front sheet-forming unit 200A, an absorbent body-forming unit 200B, a back sheet-forming unit 200C, an expandable-and-shrinkable member-bonding unit 200D, an integral bonding unit 200E and a folding unit 200F.

For conveying of the absorbent article 1 and the materials composing it, the production apparatus 200 has a machine direction MD, a cross-machine direction CD perpendicular to the machine direction MD along the conveying surface, and a thickness direction TD perpendicular to the machine direction MD and the cross-machine direction CD. However, the lengthwise direction, widthwise direction and thickness direction of the absorbent article 1 and the materials composing it are each the same as the machine direction MD, cross-machine direction CD and thickness direction TD. Thus, for the absorbent article 1 and the materials composing it as well, the machine direction MD, cross-machine direction CD and thickness direction TD are used as the lengthwise direction, widthwise direction and thickness direction, respectively.

In the front sheet-forming unit 200A, a plurality of transport rolls convey a continuous front sheet 102, as a continuous sheet for the front sheet, to a bonding roll 210. An adhesive coating applicator 201 coats the adhesive onto one side of the continuous front sheet 102 during transport. Separately, a plurality of transport rolls convey a pair of continuous leak resistant wall sheets 105, 105, as continuous sheets for the pairs of leak resistant walls, to the bonding roll 210. The bonding roll 210 compresses the continuous front sheet 102 and the pair of continuous leak resistant wall sheets 105, 105 sandwiched between the pair of bonding rolls that are disposed mutually facing each other, forming a continuous front sheet 102 with the pair of continuous leak resistant wall sheets 105, 105 bonded with an adhesive on both outer sides in the cross-machine direction. The adhesive coating applicator 202 coats the adhesive on the other side, which is the side of the transported continuous front sheet 102 opposite the side on which the continuous leak resistant wall sheets 105, 105 are bonded. A plurality of transport rolls supply the continuous front sheet 102 to the integral bonding unit 200E (bonding rolls 240, 250).

At the absorbent body-forming unit 200B, the conveyor belt supplies an absorbent body 104 formed by an absorbent body-forming apparatus (not shown) to the integral bonding unit 200E (bonding rolls 240, 250).

In the back sheet-forming unit 200C, a plurality of transport rolls convey a continuous back sheet 103, as a continuous sheet for the back sheet, to a bonding roll 220. Separately, a plurality of transport rolls transport a continuous outer sheet 109 (with the leg expandable-and-shrinkable member 6, protrusion sections 7, 8 and to-be-connected member 12 already added) as a continuous sheet for the outer sheet, to the bonding roll 220. An adhesive coating applicator 203 coats an adhesive onto one side of the continuous outer sheet 109 during transport. The bonding roll 220 compresses the continuous outer sheet 109 and the continuous back sheet 103 sandwiched between the pair of bonding rolls that are disposed mutually facing each other, forming a continuous back sheet 103 with the continuous outer sheet 109 bonded on the back side. An adhesive coating applicator 204 coats the adhesive on the other side of the transported continuous back sheet 103 where the continuous outer sheet 109 is not bonded. A plurality of transport rolls supply the continuous back sheet 103 to the integral bonding unit 200E (bonding roll 240).

In the expandable-and-shrinkable member-bonding unit 200D, a plurality of transport rolls transport a continuous expandable-and-shrinkable member sheet 111, which is a continuous sheet for the expandable-and-shrinkable member, to a cutting apparatus 230. The cutting apparatus 230 cuts the continuous expandable-and-shrinkable member sheet 111 into prescribed lengths with a cutter. A widening/attaching apparatus 400 receives the cut fragments, i.e. the expandable-and-shrinkable member 11, while holding both end sections of the expandable-and-shrinkable member 11 in the cross-machine direction. The widening/attaching apparatus 400 expands the expandable-and-shrinkable member 11 in the cross-machine direction while rotating approximately 180°. Next, the widening/attaching apparatus 400 presses the expandable-and-shrinkable member 11 in the expanded state against the side of the continuous back sheet 103 on which the adhesive was coated, which was on the bonding roll 240, to attach it to the continuous back sheet 103. Since the continuous back sheet 103 is under tension in the machine direction MD during this time, the continuous back sheet 103 undergoes virtually no narrowing even when the expandable-and-shrinkable member 11 in the extended expanded out state in the cross-machine direction CD is attached. The bonding roll 240 moves the continuous back sheet 103 onto which the expandable-and-shrinkable member has been attached, to the integral bonding unit 200E (bonding roll 250).

At the integral bonding unit 200E, the pair of bonding rolls 240, 250 that are disposed mutually facing each other compress the continuous front sheet 102 from the front sheet-forming unit 200A, the absorbent body 104 from the absorbent body-forming unit 200B and the continuous back sheet 103 from the back sheet-forming unit 200C while sandwiching them, to form a continuous body 101a of a semi-processed absorbent article having the continuous front sheet 102, the absorbent body 104 and the continuous back sheet 103 laminated.

Next, a plurality of transport rolls transport the semi-processed continuous body 101a away from the bonding roll 240, while reducing the tension in the machine direction MD. This forms a semi-processed continuous body 101b in which the portion where the expandable-and-shrinkable member is disposed is somewhat shrunk in the cross-machine direction CD. A plurality of transport rolls transport the semi-processed continuous body 101b to the folding unit 200F.

At the folding unit 200F, a folding apparatus 300 transports the semi-processed continuous body 101b by a transport roll 303 and a conveying apparatus 310, while folding it with a pressing member 301 and a folding member 302, to form a semi-processed continuous body 101c. The semi-processed continuous body 101c is then cut into individual absorbent article product portions as the absorbent article 1.

The widening/attaching apparatus 400 of the expandable-and-shrinkable member-bonding unit 200D will now be explained.

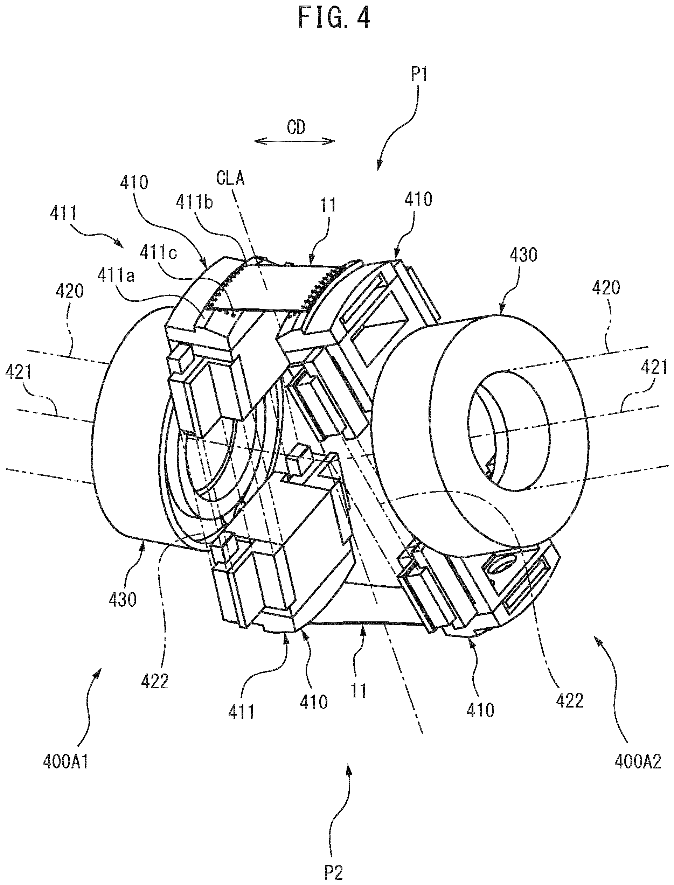

Fig. 4 is a perspective view schematically showing an example of a configuration of the widening/attaching apparatus 400. The widening/attaching apparatus 400 is an apparatus that receives a belt-shaped width-shrunk member 405 at a receiving position P1 and expands it in the cross-machine direction CD while delivering it to the continuous back sheet 103 at a delivering position P2. The widening/attaching apparatus 400 comprises a pair of widening units 400A1, 400A2 having bilateral symmetry with respect to the center line CLA. Each widening unit 400A comprises a relative moving member 430, a transport driving member 420 and a pair of transporting members 410, 410. The pair of widening units 400A1, 400A2 are set in positions so as to be mutually close at one end and mutually far at the other end (they are divergent with respect to each other). In this case, the receiving position P1 is where they are mutually close and the delivering position P2 is where they are mutually far.

The transport driving member 420 has a rotor 422 that rotates around a rotating shaft 421 as the center, the rotor 422 being mounted in a freely rotatable manner around the relative moving member 430 via a bearing. In the rotor 422, the pair of transporting members 410 are anchored at positions mutually separated 180° in the rotational direction of the rotor 422. Consequently, by rotation of the pair of transport driving members 420 at the same angular speed, rotation takes place around the relative moving member 430, with the transport members 410 facing each other on the rotors 422 of both widening units 400A1, 400A2. Each of the transport members 410 comprises a suction pad 411. The suction pad 411 is situated on the outer side surface of the transport member 410, and has a contact surface 411a with which the expandable-and-shrinkable member 11 contacts. A plurality of holes are formed in the contact surface 411a, some of the holes being suction holes 411c for suctioning of the expandable-and-shrinkable member 11 in contact with the contact surface 411a, by a suction mechanism (not shown) situated in the transport member 410, while the other holes are pinholes 411b through which pin members are inserted to anchor the expandable-and-shrinkable member 11 in contact with the contact surface 411a.

At the receiving position P1, the widening/attaching apparatus 400 receives the expandable-and-shrinkable member 11 by suctioning both end sections of the expandable-and-shrinkable member 11 by the suction holes 411c of the pair of transporting members 410. At the same time, the pin members of the pinholes 411b pierce the expandable-and-shrinkable member 11, thereby holding the expandable-and-shrinkable member 11 by the pair of transporting members 410. The widening/attaching apparatus 400 rotationally moves the pair of transporting members 410 by the pair of transport driving members 420, causing the expandable-and-shrinkable member 11 to move from the receiving position P1 to the delivering position P2. At this time, the pair of transporting members 410 rotationally move while gradually widening the distance between the suction pads 411, such that the both end sections of the expandable-and-shrinkable member 11 are pulled and expanded. Also, the widening/attaching apparatus 400 delivers the expandable-and-shrinkable member 11 by termination of the suction and pin piercing with the pair of transporting members 410 at the delivering position P2.

By adjusting the widening units 400A1, 400A2 at the widening/attaching apparatus 400, the center section in the widthwise direction CD of the expandable-and-shrinkable member 11 (for example, the portion on the inner side between the suction pads 411) can be the region with high expanding (high-expanded region) and the both end sections on both sides of the center section (for example, the portions of the suction pads 411) can be the regions with low expanding (low-expanded regions). Adjustment of the widening units 400A1, 400A2 may be, for example, adjustment of the distance between the widening units 400A1, 400A2, the degree of suction of the suction pads 411, or the presence or absence of insertion of the pin members in the pinholes 411b.

The folding apparatus 300 of the folding unit 200F will now be explained.

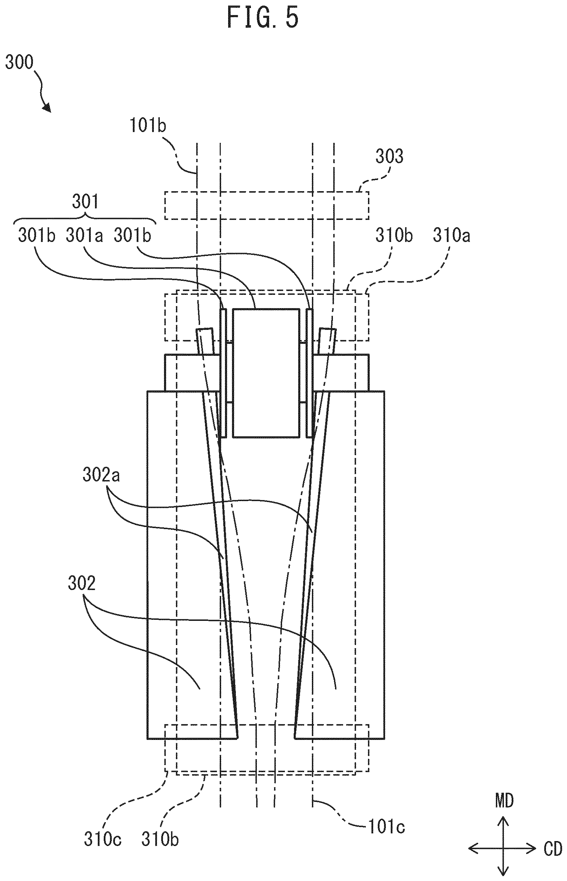

Fig. 5 is a diagram schematically showing an example of a configuration of the folding apparatus 300. The folding apparatus 300 is an apparatus that folds a continuous body at a prescribed folding position while transporting the continuous body in the machine direction MD, and it comprises a transport roll 303, a conveying apparatus 310, a pressing member 301 and a folding member 302. The semi-processed continuous body 101b will be used as an example of the continuous body.

The transport roll 303 supplies the semi-processed continuous body 101b to the pressing member 301 and the conveying apparatus 310. The pressing member 301 is disposed on the conveying apparatus 310. In the cross-machine direction CD, the length of the pressing member 301 is set to be narrower than the length of the semi-processed continuous body 101b. The pressing member 301 presses at least the center region of the semi-processed continuous body 101b toward the direction crossing the surface of the semi-processed continuous body 101b, i.e. toward the surface of the conveying apparatus 310 on which the semi-processed continuous body 101b is mounted, while transporting the semi-processed continuous body 101b in the machine direction. The conveying apparatus 310 rotates an endless belt 310b in the machine direction MD by rollers 310a, 310c, transporting the semi-processed continuous body 101b in the machine direction MD. A suction mechanism is mounted on the conveying apparatus 310. The center region of the semi-processed continuous body 101b is pressed toward the endless belt 310b by the pressing member 301, while being suctioned to the transport surface of the endless belt 310b (the surface on which the continuous body 101b is mounted). The folding member 302 guides the side section regions on both sides in the cross-machine direction CD of the semi-processed continuous body 101b that is being transported in the machine direction MD, onto the center region, by guides 302a, 302a. The suction mechanism does not need to be mounted on the conveying apparatus 310. The pressing member 301 has a pair of presser rolls 301b, 301b that press the semi-processed continuous body 101b, and a center roll 301a that is disposed between the presser rolls 301b, 301b and presses the center region of the semi-processed continuous body 101b. The position of the semi-processed continuous body 101b pressed by the presser rolls 301b, 301b becomes the folding origin, and the line drawn along the machine direction MD at that position becomes the folding line. The presser rolls 301b, 301b can be moved to any desired position between both ends and the center of the semi-processed continuous body 101b in the cross-machine direction CD. Thus, the folding line can be set with the folding apparatus 300 along the machine direction MD, at any desired position in the cross-machine direction CD. The presser rolls 301b, 301b may even be absent, in which case the function of the presser rolls 301b, 301b is carried out by both ends of the center roll 301a in the cross-machine direction CD.

At the folding apparatus 300, the transport roll 303 is disposed at a position of height h higher than the transport surface of the endless belt 310b, the width of the pressing member 301 being smaller than the width of the semi-processed continuous body 101b. This causes upward turning force to act from the transport surface of the endless belt 310b onto the pressing member 301 sides, in the side section regions of the semi-processed continuous body 101b that are not pressed against the pressing member 301. The upwardly turned side section regions are guided by the guides 302a, 302a, and fall back onto the center region. This allows folding of the side section regions (the regions connected in the machine direction MD).

For this embodiment, the width (length) of the pressing member 301 in the cross-machine direction CD is set to be narrower than the width (length) of the semi-processed continuous body 101b and narrower than the width (length) of the expandable-and-shrinkable member 11. It is preferably set to be narrower than the width (length) of the high-expanded region 11a of the expandable-and-shrinkable member 11. This allows the semi-processed continuous body 101b to be easily folded, as described below. The semi-processed continuous body 101b is folded in the folding apparatus 300 as a semi-processed continuous body 101c. The semi-processed continuous body 101c is then cut into individual absorbent article product portions as the absorbent article 1.

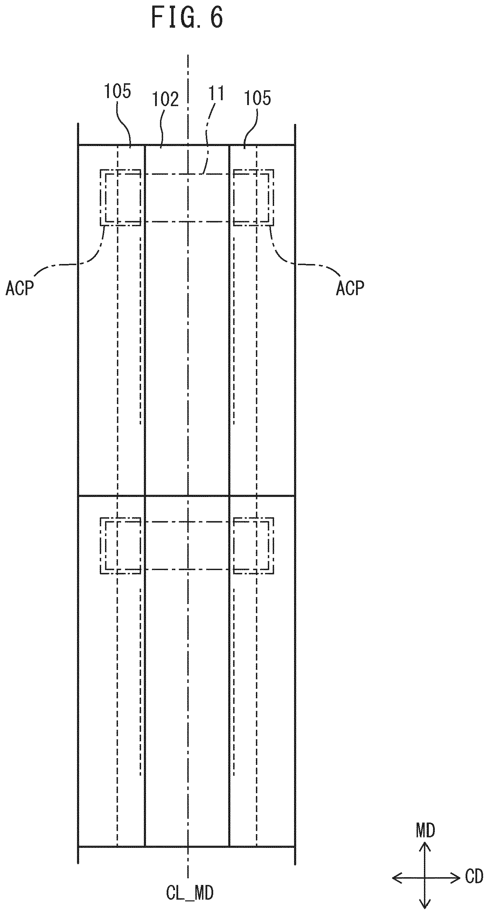

A method for producing the absorbent article of this embodiment will now be explained with reference to Fig. 3 to Fig. 5 and Fig. 6 to Fig. 11. Fig. 6 to Fig. 11 schematically show an example of the configuration of a semi-processed product, for explanation of the method for producing an absorbent article. In Fig. 6 to Fig. 11, the product runs from top to bottom in the drawing, in the machine direction MD. The production method is a method for producing an absorbent article, and it comprises a front sheet-forming step (cover sheet bonding step), an absorbent body-forming step, a back sheet-forming step, an expandable-and-shrinkable member bonding step, an integral bonding step, a shrinking step, a pressing step and a folding step.

In the front sheet-forming step (cover sheet bonding step), a continuous front sheet (second continuous sheet) 102 which is the continuous sheet for the front sheet (second sheet) is coated with an adhesive on one side by the adhesive coating applicator 201, and then supplied to the bonding roll 210. Meanwhile, a pair of continuous leak resistant wall sheets (cover sheets) 105, 105 as continuous sheets for the pairs of leak resistant walls, are supplied to the bonding roll 210. At the bonding roll 210, the continuous front sheet 102 and the pair of continuous leak resistant wall sheets 105, 105 are supplied between the pair of bonding rolls. The pair of continuous leak resistant wall sheets 105, 105 are pressed against the side of the continuous front sheet 102 on which the adhesive has been coated, thereby forming a continuous front sheet 102 having the pair of continuous leak resistant wall sheets 105, 105 bonded to both sides in the cross-machine direction. Next, by the adhesive coating applicator 202, the continuous front sheet 102 is coated with an adhesive on the other side which is the side opposite the side on which the continuous leak resistant wall sheets 105, 105 have been bonded, and then supplied to the integral bonding unit 200E (bonding rolls 240, 250).

Fig. 6 schematically shows an example of the structure of bonding between the continuous front sheet 102 and the continuous leak resistant wall sheets 105, 105. The pair of continuous leak resistant wall sheets 105, 105 are bonded at both sides in the cross-machine direction CD (widthwise direction) of the continuous front sheet 102. This diagram shows in a virtual manner the positions where both end sections ACP, ACP of the expandable-and-shrinkable member 11 are situated when the continuous front sheet 102 and the continuous back sheet 103 are bonded. Thus, in the front sheet-forming step (cover sheet bonding step), when the continuous front sheet 102 and the continuous back sheet 103 are bonded, the cover sheets (leak resistant walls 5, 5) are bonded at positions corresponding to the both end sections ACP, ACP of the continuous front sheet 102, in such a manner that the both end sections ACP, ACP are covered by the cover sheets (leak resistant walls 5, 5).

In the absorbent body-forming step, an absorbent body 104 is formed at an absorbent body-forming apparatus (not shown), and supplied to the integral bonding unit 200E (bonding rolls 240, 250) by a conveyor belt.

In the back sheet-forming step, a continuous back sheet (first continuous sheet) 103 which is the continuous sheet for the back sheet (first sheet) is supplied to the bonding roll 220. Meanwhile, a continuous outer sheet 109 (with the leg expandable-and-shrinkable member 6, protrusion sections 7, 8 and to-be-connected member 12 added), which is the continuous sheet for the outer sheet, is coated with an adhesive on one side by the adhesive coating applicator 203, and supplied to the bonding roll 220. At the bonding roll 220, the continuous outer sheet 109 and the continuous back sheet 103 are supplied between a pair of bonding rolls. Also, the continuous back sheet 103 is pressed against the side of the continuous outer sheet 109 on which the adhesive has been coated to attach them both, thereby forming a continuous back sheet 103 in which the continuous outer sheet 109 is bonded to the back side. Next, by the adhesive coating applicator 204, the continuous back sheet 103 is coated with an adhesive on the other side which is the side opposite the side on which the continuous outer sheet 109 has been bonded, and then supplied to the integral bonding unit 200E (bonding roll 240) .

In the expandable-and-shrinkable member bonding step, a continuous expandable-and-shrinkable member sheet 111 which is the continuous sheet for the expandable-and-shrinkable members, is supplied to the cutting apparatus 230 and the tip sections in the machine direction MD are cut to a prescribed length by the cutter of the cutting apparatus 230. Each cut fragment, i.e. the expandable-and-shrinkable member 11, is held at the both end sections in the cross-machine direction CD by the widening/attaching apparatus 400, and received by the widening/attaching apparatus 400. The expandable-and-shrinkable member 11 is rotated approximately 180° while the both end sections in the cross-machine direction CD are expanded in the cross-machine direction by the widening/attaching apparatus 400, and is pressed against the adhesive-coated surface of the continuous back sheet (first continuous sheet) 103 on the bonding roll 240 after the back sheet-forming step in the expanded state, and is attached to the continuous back sheet 103. The continuous back sheet 103 is under tension in the machine direction during this time, and the continuous back sheet 103 undergoes virtually no narrowing even when the expandable-and-shrinkable member 11 in the extended expanded out state in the cross-machine direction CD is attached. The continuous back sheet 103 onto which the expandable-and-shrinkable member 11 has been attached moves over the bonding roll 240 to the integral bonding unit 200E (bonding roll 250).

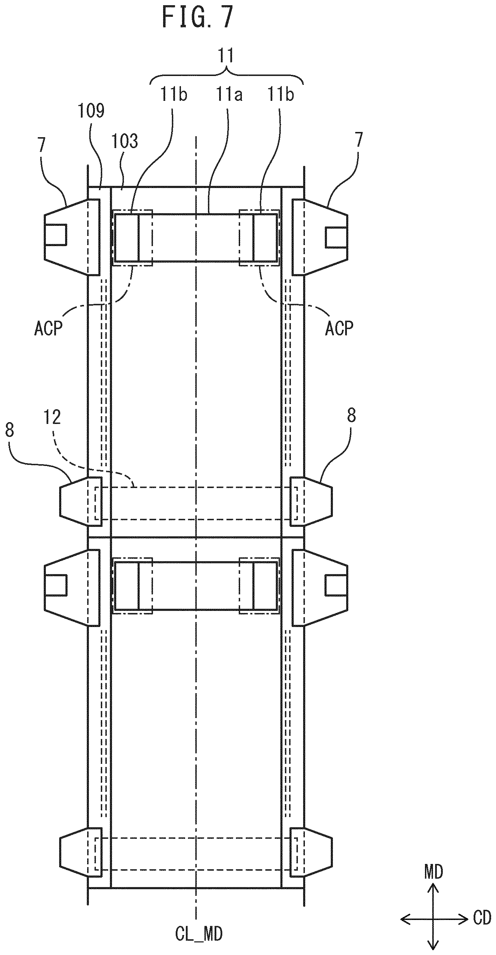

Fig. 7 schematically shows an example of the structure of bonding between the continuous back sheet 103 and the expandable-and-shrinkable member 11. The expandable-and-shrinkable member 11 that is expandable and shrinkable in the expansion-and-shrink direction is bonded to the continuous back sheet 103 with the center section of the expandable-and-shrinkable member 11 in the expansion-and-shrink direction in the high-expanded region 11a and the both sides in the low-expanded regions 11b, 11b, so that the expansion-and-shrink direction extends along the cross-machine direction CD (widthwise direction). In this case, the both end sections ACP, ACP generally corresponding to the low-expanded regions 11b, 11b of the expandable-and-shrinkable member 11 are the regions to be covered by the cover sheet (leak resistant walls 5, 5).

When the expandable-and-shrinkable member 11 is thus attached to the continuous back sheet 103, the center section of the expandable-and-shrinkable member 11 is the high-expanded region 11a and the both sides are the low-expanded regions 11b, 11b, such that internal stress that tends to create concavity in the surface of the expandable-and-shrinkable member 11 side of the laminate of the expandable-and-shrinkable member 11 and the continuous back sheet 103 can be reduced at the both end sections ACP, ACP of the expandable-and-shrinkable member 11. In other words, the internal stress by which the laminate tends to warp around to the expandable-and-shrinkable member 11 side can be reduced. This makes it possible to prevent detachment of bonding between the both end sections ACP, ACP of the expandable-and-shrinkable member 11, and the continuous back sheet 103.

In the integral bonding step, the continuous front sheet (second continuous sheet) 102 on which the continuous leak resistant wall sheets (cover sheets) 105, 105 are bonded from the front sheet-forming step (cover sheet bonding step), the absorbent body 104 from the absorbent body-forming step, and the continuous back sheet (first continuous sheet) 103 on which the expandable-and-shrinkable member 11 is attached from the expandable-and-shrinkable member bonding step, are transported between the pair of bonding rolls 240, 250. Also, the continuous front sheet 102, the absorbent body 104 and the continuous back sheet 103 are sandwiched, compressed and bonded between the pair of bonding rolls 240, 250. A semi-processed continuous body 101a of the absorbent article is thus formed comprising the continuous front sheet 102, the absorbent body 104 and the continuous back sheet 103.

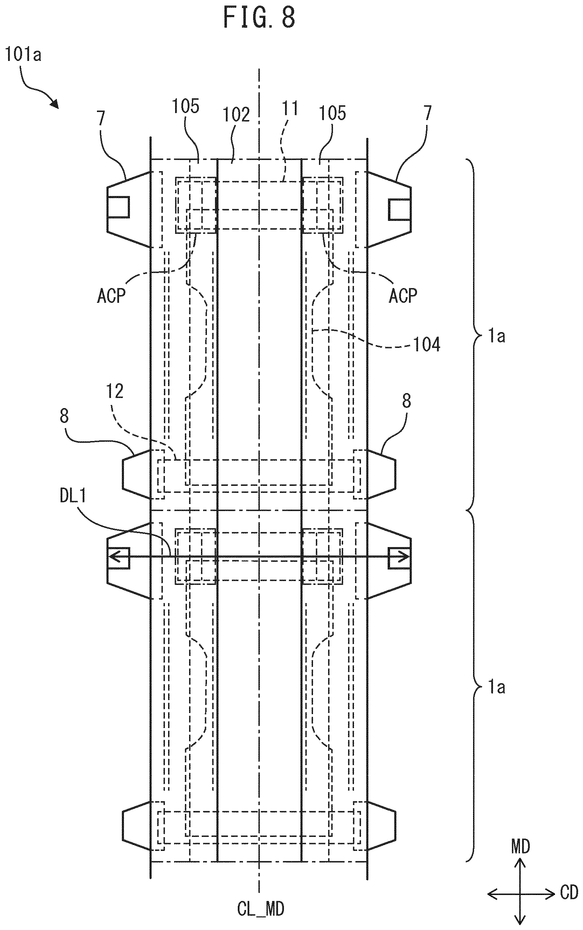

Fig. 8 schematically shows an example of the structure of a semi-processed continuous body 101a. The semi-processed continuous body 101a is composed of a plurality of semi-processed products 1a connected in the machine direction. One semi-processed product 1a has virtually no wrinkles 11S in the expandable-and-shrinkable member 11, and has the same structure as the absorbent article 1 shown in Fig. 1. In this case, the continuous front sheet 102 and the continuous back sheet 103 are bonded while the both end sections ACP, ACP of the expandable-and-shrinkable member 11 are covered by the pair of leak resistant walls 5, 5. Stated differently, the continuous front sheet 102 and the continuous back sheet 103 are bonded and the both end sections ACP, ACP of the expandable-and-shrinkable member 11 are covered by the pair of leak resistant walls 5, 5 (cover sheet).

In this case, the both end sections ACP, ACP of the expandable-and-shrinkable member 11 covered by the pair of leak resistant walls 5, 5 have increased thickness in the thickness direction (TD) compared to when they are not covered by the pair of leak resistant walls 5, 5. Thus, when the continuous front sheet 102 and continuous back sheet 103 are bonded, the both end sections ACP, ACP of the expandable-and-shrinkable member 11 are more strongly pressed. That is, the bonded sections between the both end sections ACP, ACP of the expandable-and-shrinkable member 11 and the continuous back sheet 103 are compressed more forcefully. Consequently, bonding between the both end sections ACP, ACP of the expandable-and-shrinkable member 11 and the continuous back sheet 103, which can potentially become weakened, can be reinforced by the compression. In addition, even if unnatural irregular shapes or the like have been generated at the both end sections ACP, ACP of the expandable-and-shrinkable member 11, the compression can minimize the degree of irregularities and improve the product appearance. Furthermore, even if irregular shapes and the like are generated at the both end sections ACP, ACP of the expandable-and-shrinkable member 11, the irregular shapes can be rendered less visible from the exterior by the pair of leak resistant walls 5, 5. This allows the appearance of the product to be improved.

In the cross-machine direction CD, the maximum length at the portion of the semi-processed product 1a including the expandable-and-shrinkable member 11 (including the lengths of the protruding sections of the pair of protrusion sections 7, 7) is DL1. In this case, the expandable-and-shrinkable member 11 of the semi-processed product 1a undergoes virtually no shrink by tension on the semi-processed continuous body 101a. Thus, the expandable-and-shrinkable member 11 is disposed in the expanded state in the cross-machine direction CD, but shrink is minimal.

In the shrinking step, the semi-processed continuous body 101a separates from the bonding roll 240 and tension in the machine direction MD is reduced (for example, the rotational speed of the transport roll is reduced). This allows the expandable-and-shrinkable member 11 expanded in the cross-machine direction CD to shrink, thereby allowing the expandable-and-shrinkable member 11 to be shrunk in the cross-machine direction CD. In response, the portions of the semi-processed product 1a including the expandable-and-shrinkable member 11 can also shrink in the cross-machine direction CD. As a result, a semi-processed continuous body 101b is formed in which the portions including the expandable-and-shrinkable member 11 are shrunk in the cross-machine direction CD.

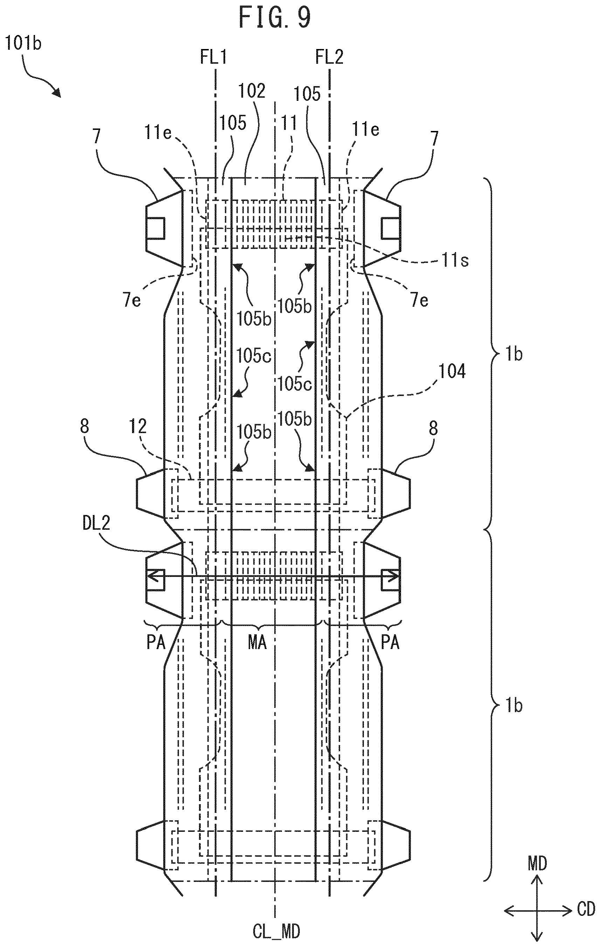

Fig. 9 schematically shows an example of the structure of a semi-processed continuous body 101b. The semi-processed continuous body 101b is composed of a plurality of semi-processed products 1b connected in the machine direction. One semi-processed product 1b has basically the same structure as the semi-processed product 1a shown in Fig. 8. Compared to the semi-processed product 1a, however, the portions of the semi-processed product 1b including the expandable-and-shrinkable member 11 are shrunk in the cross-machine direction CD (widthwise direction). That is, the reduced tension on the semi-processed continuous body 101a causes the expandable-and-shrinkable member 11 disposed in an expanded state in the cross-machine direction CD, to be shrunk in the cross-machine direction CD of the semi-processed product 1b. At the portions of the semi-processed continuous body 101b including the expandable-and-shrinkable member 11, the maximum length in the cross-machine direction CD (including the length of the protruding sections of the pair of protrusion sections 7, 7) DL2 is smaller than the length DL1 before shrink of the expandable-and-shrinkable member 11 (Fig. 6) (DL2 < DL1). At the same time, a plurality of wrinkles 11S are formed at the portions of the semi-processed continuous body 101b including the expandable-and-shrinkable member 11. The semi-processed continuous body 101b is transported by the folding unit 200F.

In this shrinking step as well, unnatural irregular shapes or the like may potentially be generated at the both end sections ACP, ACP of the expandable-and-shrinkable member 11. In the integral bonding step, however, bonding between the both end sections ACP, ACP of the expandable-and-shrinkable member 11 and the continuous back sheet 103, which can potentially be lowered, is reinforced by the compression, and therefore generation of irregular shapes and the like can be minimized. Also, even if irregular shapes and the like are generated, the both end sections ACP, ACP of the expandable-and-shrinkable member 11 are covered by the pair of leak resistant walls 5, 5, and therefore the irregular shapes can be rendered less visible from the exterior. This allows the appearance of the product to be improved.

In the following pressing step, the semi-processed continuous body 101b with the expandable-and-shrinkable member 11 shrunk is transported in the machine direction MD, while a center region MA of the semi-processed product 1b in the cross-machine direction CD is pressed in the direction toward the mounting surface of the semi-processed product 1b (the transport surface of the endless belt 310b), with the pressing member 301 whose length in the cross-machine direction CD is smaller than the length of the expandable-and-shrinkable member 11 in the cross-machine direction CD. As a result, the region of the expandable-and-shrinkable member 11 with the plurality of wrinkles 11S becomes pressed.

In the folding step, the semi-processed continuous body 101b is transported in the machine direction MD while a pair of side section regions PA, PA on the outer sides of the center region MA of the semi-processed product 1b in the cross-machine direction CD are raised in the direction opposite from the direction toward the mounting surface of the semi-processed product 1b by the folding member 302 (guides 302a, 302a) on a pair of folding lines FL1, FL2 extending along the machine direction MD, and each is folded upward above the center region MA. A semi-processed continuous body 101c is thus formed. However, the pair of folding lines FL1, FL2 are set so as to extend along the machine direction MD, to the inner side from both edge lines 11e, 11e of the expandable-and-shrinkable member 11 in the cross-machine direction CD. They are preferably set so as to extend along the machine direction MD, to the inner side from the edge lines 7e, 7e on the inner side in the widthwise direction CD of the pair of protrusion sections 7, 7. The pressing step and the folding step may be carried out in a temporally overlapping manner, or the folding step may be carried out after the pressing step.

The semi-processed product 1b has the pair of protrusion sections 7, 7 that are discontinuous in the machine direction MD, formed so as to protrude to the outer sides at both ends in the cross-machine direction CD (widthwise direction), and at the folding apparatus 300, the discontinuous pair of protrusion sections 7, 7 can potentially be difficult to fold in a stable manner. According to this embodiment, however, the use of the expandable-and-shrinkable member 11 reduces the length DL2 of the portion of the semi-processed continuous body 101b including the expandable-and-shrinkable member 11 (the portion between the protrusion sections 7, 7) in the cross-machine direction CD, thereby relatively reducing the moment of inertia around the folding lines FL1, FL2. This can reduce the force necessary to rotate the portion further outward than the folding lines FL1, FL2, around the folding lines FL1, FL2 as the center of rotation. As a result, the discontinuous protrusion sections 7, 7 can be easily raised and folded. (2) The plurality of wrinkles 11S are formed in the portion of the semi-processed continuous body 101b including the expandable-and-shrinkable member 11 (the portion between the protrusion sections 7, 7), and as some of the wrinkles 11S are collapsed by the pressing member 301, the side section regions PA that are not pressed rise in the opposite direction. Consequently, the protrusion sections 7, 7 disposed on both sides of the side section region PA in the cross-machine direction CD can also easily rise and be easily folded.

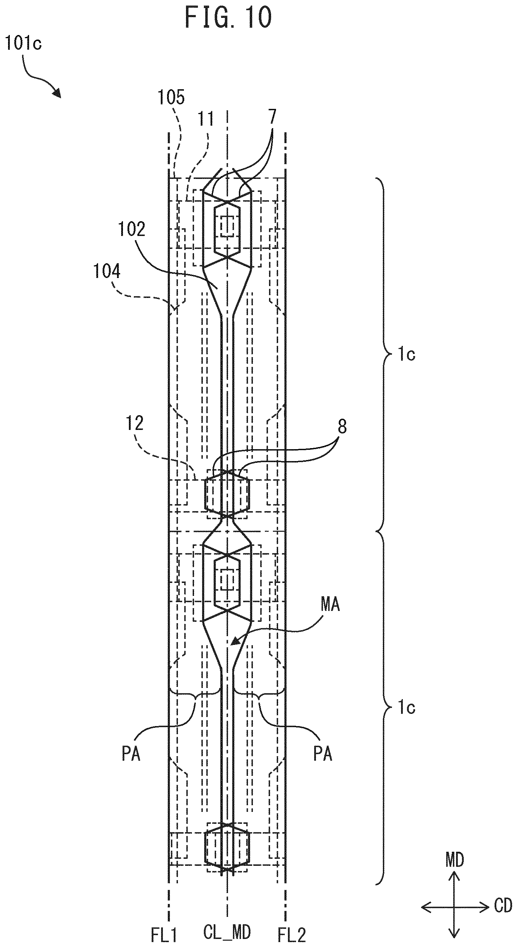

Fig. 10 schematically shows an example of the structure of a semi-processed continuous body 101c. The semi-processed continuous body 101c is composed of a plurality of semi-processed products 1c connected in the machine direction. One semi-processed product 1c has basically the same structure as the semi-processed product 1b shown in Fig. 7. However, compared to the semi-processed product 1b, the pair of side section regions PA, PA of the semi-processed product 1c are each folded upward over the center region MA.

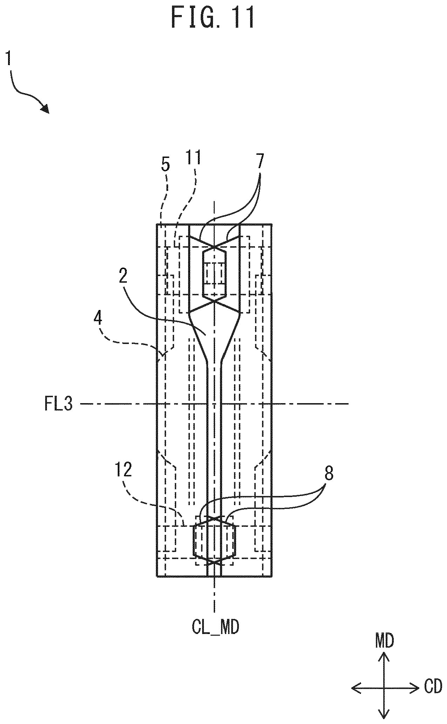

In the folding step, the semi-processed continuous body 101c is further cut into individual absorbent article product portions as the absorbent article 1. Fig. 11 schematically shows an example of the structure of the absorbent article 1 cut into a product portion. The cut absorbent article 1 has the same structure as the semi-processed product 1c of Fig. 8. Next, the absorbent article 1 is folded in the machine direction MD at a folding line FL3 that extends through the center section in the machine direction MD and extends along the cross-machine direction CD.

The absorbent article is produced in this manner.

Thus, in this method for producing an absorbent article, the bonding performance between the expandable-and-shrinkable member 11 at the both end sections ACP, ACP of the expandable-and-shrinkable member 11, and the continuous back sheet 103, i.e. the back sheet 3, is improved, while generation of irregular shapes and the like is also minimized, and even if irregular shapes and the like are generated the product appearance can be improved.

In the expandable-and-shrinkable member bonding step, the adhesive may be coated on the continuous back sheet 103 or coated on the expandable-and-shrinkable member 11. Also, the adhesive is preferably a hot-melt adhesive. In such cases, even if irregular shapes or the like are generated at the edges ACP of the expandable-and-shrinkable member 11, the hot-melt adhesive is present between the irregular shapes and the continuous back sheet 103, such that the irregular shapes are poorly visible. This allows the appearance of the product to be improved.

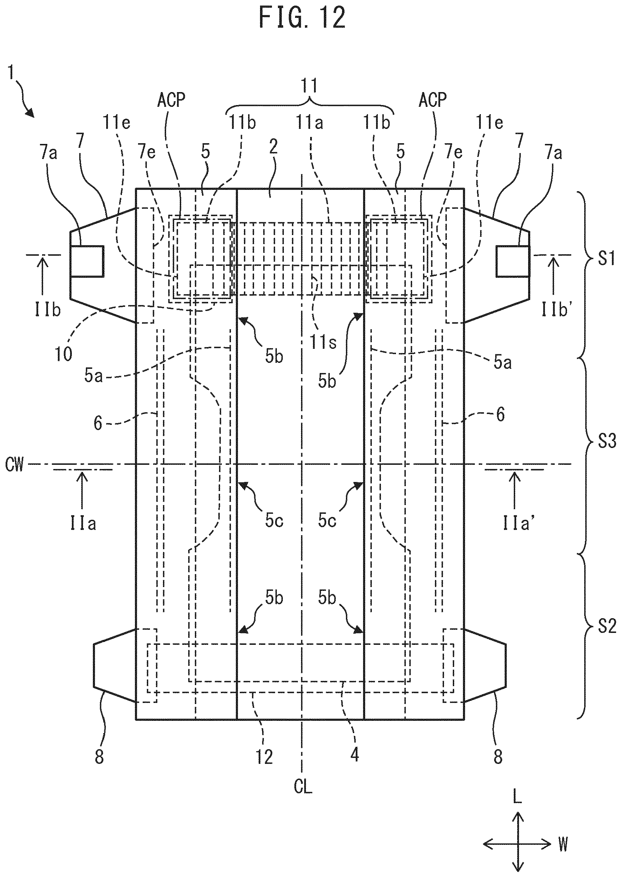

A production apparatus for an absorbent article according to another embodiment will now be described. This embodiment will be explained mainly in terms of its differences from the embodiment of Fig. 1 to Fig. 11. Fig. 12 is a diagram showing an absorbent article 1 (disposable diaper), and specifically it is a plan view of the absorbent article 1 in the deployed spread-out state. For this embodiment, as sheets covering the both end sections ACP, ACP of the expandable-and-shrinkable member 11, there are disposed cover sheets 10, 10 having essentially the same shapes as the both end sections ACP, ACP, in a manner covering the both end sections ACP, ACP. The cover sheets 10, 10 may be disposed on the expandable-and-shrinkable member 11 side of the front sheet 2, or disposed between the front sheet 2 and the leak resistant walls 5, 5 (as in Fig. 12), or disposed on the skin side of the leak resistant walls 5, 5. The joints 5b of the leak resistant walls 5, 5 may be made smaller in the lengthwise direction L, so as not to overlap with the expandable-and-shrinkable member 11 and the cover sheets 10, 10.

The step of bonding the cover sheets 10, 10 in the production method involves carrying out a front sheet-forming step (cover sheet bonding step) with the front sheet-forming unit 200A. That is, either the cover sheets 10, 10 are bonded to the continuous front sheet 102 before bonding the pair of continuous leak resistant wall sheets 105, 105 to the continuous front sheet 102, or the cover sheets 10, 10 are bonded to the pair of continuous leak resistant wall sheets 105, 105 after the pair of continuous leak resistant wall sheets 105, 105 have been bonded to the continuous front sheet 102. The bonding position is the position where the both end sections ACP, ACP are covered by the cover sheets 10, 10 when the continuous front sheet 102 and the continuous back sheet 103 are bonded.

In this case as well, the same effect can be obtained as with the embodiment described above. Moreover, when the pair of leak resistant walls 5, 5 and the cover sheets 10, 10 are overlapping, the both end sections ACP of the expandable-and-shrinkable member 11 are less likely to separate, irregular shapes and the like are less likely to be generated and irregular shapes and the like can be more easily hidden.

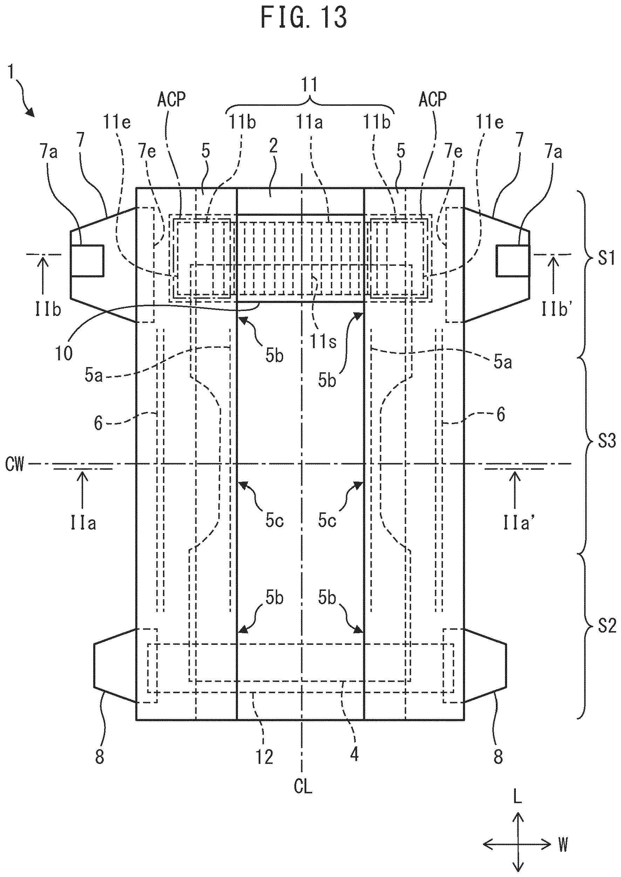

A production apparatus for an absorbent article according to yet another embodiment will now be described. This embodiment will be explained mainly in terms of its differences from the embodiment of Fig. 12. Fig. 13 is a diagram showing an absorbent article 1 (disposable diaper), and specifically it is a plan view of the absorbent article 1 in the deployed spread-out state. For this embodiment, as sheets covering the both end sections ACP, ACP of the expandable-and-shrinkable member 11, there is disposed a special cover sheet 10 having essentially the same shape as the expandable-and-shrinkable member 11, in a manner covering the entire expandable-and-shrinkable member 11 including the both end sections ACP, ACP. In this case as well, the same effect is obtained as in Fig. 12.

In another preferred embodiment of the method for producing the absorbent article, the front sheet-forming step (cover sheet bonding step) may include a step of bonding the continuous leak resistant wall sheets (cover sheets) 105, 105 to the continuous front sheet 102 by a heat sealing method or an ultrasonic method. Even if irregular shapes and the like are generated at the edges ACP of the expandable-and-shrinkable member 11, the cover sheet and the irregular shapes that have been bonded by a heat sealing method or by ultrasonic waves become overlapping, and the irregular shapes can be rendered poorly visible. Alternatively, according to another preferred embodiment, there is further provided a step of forming embossed sections along the lengthwise direction, extending through the both end sections ACP, ACP of the expandable-and-shrinkable member 11, i.e. an embossing step. Even if irregular shapes are produced on the edges ACP of the expandable-and-shrinkable member 11, the embossed sections and the irregular shapes overlap, so that the irregular shapes can be poorly visible. Alternatively, according to another preferred embodiment, the front sheet-forming step (cover sheet bonding step) may include a step in which the continuous leak resistant wall sheets (cover sheets) 105, 105 formed of a colored or opaque material, or a material of the same color as the expandable-and-shrinkable member 11, are bonded to the continuous front sheet 102. Even if irregular shapes or the like are generated at the edges ACP of the expandable-and-shrinkable member 11, the cover sheet which is colored, opaque or of the same color as the expandable-and-shrinkable member 11 can make the irregular shapes poorly visible.

For this embodiment, the cover sheet (leak resistant wall 5, cover sheet 10) is bonded to the front sheet 2 (continuous front sheet 102), but the present invention is not limited to this example. The cover sheet (for example the cover sheet 10) may be bonded to the back sheet 3 (continuous back sheet 103) and/or expandable-and-shrinkable member 11 so as to cover the both end sections ACP, ACP. In this case, as the bonding step for the cover sheet in the production method, there is added a step of attaching the cover sheet to the continuous back sheet 103 to which the expandable-and-shrinkable member 11 has been bonded in the expandable-and-shrinkable member bonding step, on the bonding roll 240. Since the cover sheet directly covers the both end sections ACP, ACP, the effect of the cover sheet can be even higher.

For this embodiment, the cover sheet (leak resistant wall 5, cover sheet 10) is bonded to the continuous front sheet 102 or continuous back sheet 103 before being bonded to the continuous front sheet 102 and the continuous back sheet 103, but the present invention is not limited to this example. The cover sheet (for example, the cover sheet 10) may be bonded to the pair of continuous leak resistant wall sheets 105, 105 and/or the continuous front sheet 102 so as to cover the both end sections ACP, ACP, after the continuous front sheet 102 and the continuous back sheet 103 have been bonded.

The absorbent article of the present invention is not limited to the embodiments described above and can incorporate appropriate combinations and modifications without departing from the scope of the object of the present invention.

### Reference Signs List

1 Absorbent article
1a, 1b, 1c Semi-processed product
11 Expandable-and-shrinkable member
101a, 101b, 101c Semi-processed continuous body 102 Continuous front sheet
103 Continuous back sheet
105 Continuous leak resistant wall sheet

## Claims

1. A method for producing an absorbent article (1) from a first continuous sheet and a second continuous sheet, the method comprising:
a cover sheet bonding step of bonding a cover sheet (105) to the second continuous sheet that comprises a second sheet (2) constituting the absorbent article, connected in a machine direction,
an expandable-and-shrinkable member bonding step of bonding an expandable-and-shrinkable member (11) that is expandable and shrinkable in an expansion-and-shrink direction so that the expansion-and-shrink direction is along a widthwise direction of the first sheet, with a center section (11a) of the expandable-and-shrinkable member in the expansion-and-shrink direction in a high-expanded state and both end sections (11b, 11b) on both sides of the center section in a low-expanded state, while transporting the first continuous sheet that comprises a first sheet (3) constituting the absorbent article, connected in the machine direction, in the machine direction; and
an integral bonding step of successively bonding the first sheet and the second sheet, while transporting each of the first continuous sheet and the second continuous sheet in the machine direction,
wherein the cover sheet bonding step includes a cover sheet bonding step of bonding the cover sheet at locations corresponding to the both end sections of the expandable-and-shrinkable member so that the both end sections of the expandable-and-shrinkable member are covered by the cover sheet with the first sheet and the second sheet in a bonded state, and includes a step of situating a pair of leak resistant walls extending along the machine direction and separated in the widthwise direction of the first sheet or the second sheet on the first sheet or the second sheet in such a manner that edges of the leak resistant walls in the machine direction cover the both end sections of the expandable-and-shrinkable member as the cover sheet.

2. The method for producing an absorbent article according to claim 1, wherein the expandable-and-shrinkable member bonding step includes a step of coating a hot-melt adhesive onto at least one of the first sheet and the expandable-and-shrinkable member.

3. The method for producing an absorbent article according to claim 1 or 2, wherein the cover sheet bonding step includes a step of bonding the cover sheet to the second continuous sheet by a heat sealing method.

4. The method for producing an absorbent article according to claim 1 or 2, wherein the cover sheet bonding step includes a step of bonding the cover sheet to the second continuous sheet by an ultrasonic method.

5. The method for producing an absorbent article according to any one of claims 1 to 4, further comprising a step of forming embossed sections along a lengthwise direction extending through both end sections of the expandable-and-shrinkable member.

6. The method for producing an absorbent article according to any one of claims 1 to 5, wherein the cover sheet bonding step includes a step of bonding the cover sheet formed of a colored material to the second continuous sheet or the first continuous sheet.

7. The method for producing an absorbent article according to claim 6, wherein the cover sheet bonding step includes a step of bonding the cover sheet formed of a material of the same color as the expandable-and-shrinkable member to the second continuous sheet or the first continuous sheet.

## Patentansprüche

1. Verfahren für die Herstellung eines absorbierenden Artikels (1) aus einer ersten fortlaufenden Lage und einer zweiten fortlaufenden Lage, wobei das Verfahren Folgendes umfasst:
einen Decklagenbindungsschritt zur Bindung einer Decklage (105) an die zweite fortlaufende Lage, die eine zweite Lage (2) umfasst, die den absorbierenden Artikel bildet, verbunden in einer Maschinenlaufrichtung,
einen Bindungsschritt des erweiterbaren und schrumpfbaren Elements zur Bindung eines erweiterbaren und schrumpfbaren Elements (11), das in einer Erweiterung-und-Schrumpfung-Richtung erweiterbar und schrumpfbar ist, sodass die Erweiterung-und-Schrumpfung-Richtung entlang einer Breitenrichtung der ersten Lage verläuft, mit einem mittleren Abschnitt (11a) des erweiterbaren und schrumpfbaren Elements in der Erweiterung-und-Schrumpfung-Richtung in einem stark erweiterten Zustand und beiden Endabschnitten (11b, 11b) an beiden Seiten des mittleren Abschnitts in einem gering erweiterten Zustand, während die erste fortlaufende Lage, die eine erste Lage (3) umfasst, die den absorbierenden Artikel bildet, verbunden in der Maschinenrichtung, in der Maschinenlaufrichtung befördert wird; und
einen Integralbindungsschritt zur aufeinanderfolgenden Bindung der ersten Lage und der zweiten Lage, während jede der ersten fortlaufenden Lage und der zweiten fortlaufenden Lage in der Maschinenlaufrichtung befördert wird,
wobei der Decklagenbindungsschritt einen Decklagenbindungsschritt zur Bindung der Decklage an Stellen einschließt, die den beiden Endabschnitten des erweiterbaren und schrumpfbaren Elements entsprechen, sodass die beiden Endabschnitte des erweiterbaren und schrumpfbaren Elements durch die Decklage abgedeckt werden, wobei die erste Lage und die zweite Lage in einem gebundenen Zustand vorliegen, und einen Schritt des Platzierens eines Paars von auslaufsicheren Wänden, die sich entlang der Maschinenlaufrichtung erstrecken und in der Breitenrichtung der ersten Lage oder der zweiten Lage getrennt sind, auf der ersten Lage oder der zweiten Lage in einer derartigen Weise einschließt, dass Ränder der auslaufsicheren Wände in der Maschinenlaufrichtung die beiden Endabschnitte des erweiterbaren und schrumpfbaren Elements als Decklage abdecken.

2. Verfahren für die Herstellung eines absorbierenden Artikels nach Anspruch 1, wobei der Bindungsschritt des erweiterbaren und schrumpfbaren Elements einen Schritt der Beschichtung eines Schmelzklebers auf mindestens eines von der ersten Lage und dem erweiterbaren und schrumpfbaren Element einschließt.

3. Verfahren für die Herstellung eines absorbierenden Artikels nach Anspruch 1 oder 2, wobei der Decklagenbindungsschritt einen Schritt zur Bindung der Decklage an die zweite fortlaufende Lage durch ein Heißsiegelverfahren einschließt.

4. Verfahren für die Herstellung eines absorbierenden Artikels nach Anspruch 1 oder 2, wobei der Decklagenbindungsschritt einen Schritt zur Bindung der Decklage an die zweite fortlaufende Lage durch ein Ultraschallverfahren einschließt.

5. Verfahren für die Herstellung eines absorbierenden Artikels nach einem der Ansprüche 1 bis 4, das weiter einen Schritt zur Ausbildung von geprägten Abschnitten entlang einer Längsrichtung umfasst, die sich durch beide Endabschnitte des erweiterbaren und schrumpfbaren Elements erstrecken.

6. Verfahren für die Herstellung eines absorbierenden Artikels nach einem der Ansprüche 1 bis 5, wobei der Decklagenbindungsschritt einen Schritt zur Bindung der Decklage, die aus einem farbigen Material ausgebildet ist, an die zweite fortlaufende Lage oder die erste fortlaufende Lage einschließt.

7. Verfahren für die Herstellung eines absorbierenden Artikels nach Anspruch 6, wobei der Decklagenbindungsschritt einen Schritt zur Bindung der Decklage, die aus einem Material der gleichen Farbe wie das erweiterbare und schrumpfbare Element ausgebildet ist, an die zweite fortlaufende Lage oder die erste fortlaufende Lage einschließt.

## Revendications

1. Procédé de production d'un article absorbant (1) à partir d'une première feuille continue et d'une deuxième feuille continue, le procédé comportant :
une étape de liaison de feuille de couverture consistant à lier une feuille de couverture (105) sur la deuxième feuille continue qui comporte une deuxième feuille (2) constituant l'article absorbant, connectée dans une direction allant dans le sens machine,
une étape de liaison d'élément en mesure de s'étendre et de rétrécir consistant à lier un élément en mesure de s'étendre et de rétrécir (11) qui est en mesure de s'étendre et de rétrécir dans une direction allant dans le sens de l'extension et du rétrécissement de telle sorte que la direction allant dans le sens de l'extension et du rétrécissement va le long d'une direction allant dans le sens de la largeur de la première feuille, avec une section centrale (11a) de l'élément en mesure de s'étendre et de rétrécir dans la direction allant dans le sens de l'extension et du rétrécissement dans un état hautement étendu et deux sections d'extrémité (11b, 11b) des deux côtés de la section centrale dans un état faiblement étendu, tout en transportant la première feuille continue qui comporte une première feuille (3) constituant l'article absorbant, connectée dans la direction allant dans le sens machine, dans la direction allant dans le sens machine ; et
une étape de liaison intégrale consistant à lier de manière successive la première feuille et la deuxième feuille, tout en transportant chacune de la première feuille continue et de la deuxième feuille continue dans la direction allant dans le sens machine,
dans lequel l'étape de liaison de feuille de couverture comprend une étape de liaison de feuille de couverture consistant à lier la feuille de couverture au niveau d'emplacements qui correspondent auxdites deux sections d'extrémité de l'élément en mesure de s'étendre et de rétrécir de telle sorte que lesdites deux sections d'extrémité de l'élément en mesure de s'étendre et de rétrécir sont recouvertes par la feuille de couverture avec la première feuille et la deuxième feuille dans un état lié, et comprend une étape consistant à positionner une paire de parois résistantes aux fuites s'étendant le long de la direction allant dans le sens machine et séparées dans la direction allant dans le sens machine de la première feuille ou de la deuxième feuille sur la première feuille ou la deuxième feuille d'une telle manière que les bords des parois résistantes aux fuites dans la direction allant dans le sens machine recouvrent lesdites deux sections d'extrémité de l'élément en mesure de s'étendre et de rétrécir comme la feuille de couverture.

2. Procédé de production d'un article absorbant selon la revendication 1, dans lequel l'étape de liaison d'élément en mesure de s'étendre et de rétrécir comprend une étape consistant à enduire un adhésif thermofusible sur au moins l'un parmi la première feuille et l'élément en mesure de s'étendre et de rétrécir.

3. Procédé de production d'un article absorbant selon la revendication 1 ou la revendication 2, dans lequel l'étape de liaison de feuille de couverture comprend une étape consistant à lier la feuille de couverture à la deuxième feuille continue par un procédé de thermoscellage.

4. Procédé de production d'un article absorbant selon la revendication 1 ou la revendication 2, dans lequel l'étape de liaison de feuille de couverture comprend une étape consistant à lier la feuille de couverture à la deuxième feuille continue par un procédé à ultrasons.

5. Procédé de production d'un article absorbant selon l'une quelconque des revendications 1 à 4, comportant par ailleurs une étape consistant à former des sections gaufrées le long d'une direction allant dans le sens de la longueur s'étendant au travers des deux sections d'extrémité de l'élément en mesure de s'étendre et de rétrécir.

6. Procédé de production d'un article absorbant selon l'une quelconque des revendications 1 à 5, dans lequel l'étape de liaison de feuille de couverture comprend une étape consistant à lier la feuille de couverture formée à partir d'un matériau de couleur à la deuxième feuille continue ou à la première feuille continue.

7. Procédé de production d'un article absorbant selon la revendication 6, dans lequel l'étape de liaison de feuille de couverture comprend une étape consistant à lier la feuille de couverture formée à partir d'un matériau de la même couleur que l'élément en mesure de s'étendre et de rétrécir à la deuxième feuille continue ou à la première feuille continue.
